# EUROPÄISCHE PATENTANMELDUNG

(11) **EP 2 993 432 A1**
(43) Veröffentlichungstag der Anmeldung: **09.03.2016**
(21) Anmeldenummer: 15002354.7
(22) Anmeldetag: 06.08.2015
(51) Int. Cl.: F25J 3/04, G01N 1/00, G01N 33/00

(54) **VERFAHREN ZUR TIEFTEMPERATURZERLEGUNG VON LUFT UND LUFTZERLEGUNGSANLAGE**

(30) Priorität: 02.09.2014 EP 14003025
(71) Anmelder: Linde Aktiengesellschaft, 80331 München (DE)
(72) Erfinder: Meilinger, Matthias, 82515 Wolfratshausen (DE)
(74) Vertreter: Imhof, Dietmar

(57) **Zusammenfassung**

Die Erfindung betrifft ein Verfahren zur Tieftemperaturzerlegung von Luft, bei dem in einem Destillationssäulensystem (10) einer Luftzerlegungsanlage (100) ein in einem Verdampfungsraum vorliegendes Flüssigkeitsvolumen mittels einer tiefkalten Flüssigkeit gespeist wird und bei dem kontinuierlich ein Teil des Flüssigkeitsvolumens durch Verdampfen in die Gasphase überführt wird, wobei die tiefkalte Flüssigkeit neben Sauerstoff schwerer als Sauerstoff siedende Komponenten, darunter Xenon, enthält. Der Gehalt an Xenon in der tiefkalten Flüssigkeit wird bestimmt und als ein Maß für eine Anreicherung der schwerer als Sauerstoff siedenden Komponenten in der tiefkalten Flüssigkeit verwendet. Eine entsprechende Luftzerlegungsanlage ist ebenfalls Gegenstand der vorliegenden Erfindung.

## Beschreibung

Die vorliegende Erfindung betrifft ein Verfahren zur Tieftemperaturzerlegung von Luft und eine entsprechende Luftzerlegungsanlage gemäß den Oberbegriffen der unabhängigen Patentansprüche.

### Stand der Technik

Die Herstellung von Luftprodukten in flüssigem oder gasförmigem Zustand durch Tieftemperaturzerlegung von Luft in Luftzerlegungsanlagen ist bekannt und beispielsweise bei H.-W. Häring (Hrsg.), Industrial Gases Processing, Wiley-VCH, 2006, insbesondere Abschnitt 2.2.5, "Cryogenic Rectification", beschrieben.

Luftzerlegungsanlagen weisen Destillationssäulensysteme auf, die beispielsweise als Zweisäulensysteme, insbesondere als klassische Linde-Doppelsäulensysteme, aber auch als Drei- oder Mehrsäulensysteme ausgebildet sein können. Neben den Destillationssäulen zur Gewinnung von Stickstoff und/oder Sauerstoff in flüssigem und/oder gasförmigem Zustand (beispielsweise flüssigem Sauerstoff, LOX, gasförmigem Sauerstoff, GOX, flüssigem Stickstoff, LIN und/oder gasförmigem Stickstoff, GAN), also den Destillationssäulen zur Stickstoff-Sauerstoff-Trennung, können Destillationssäulen zur Gewinnung weiterer Luftkomponenten, insbesondere der Edelgase Krypton, Xenon und/oder Argon, vorgesehen sein.

Die vorliegende Erfindung ist insbesondere zum Einsatz in Luftzerlegungsanlagen vorgesehen, bei denen dem verwendeten Destillationssäulensystem zur Stickstoff-Sauerstoff-Trennung sauerstoffreiche Ströme überwiegend oder ausschließlich in gasförmigem Zustand entnommen werden. Die Erfindung kann aber auch in Luftzerlegungsanlagen zum Einsatz kommen, bei denen zur Bereitstellung sauerstoffreicher Produkte dem Destillationssäulensystem flüssige Ströme entnommen werden, beispielsweise in Luftzerlegungsanlagen mit Innenverdichtung, sofern hier eine nachfolgend erläuterte Anreicherung schwerer als Sauerstoff siedender Komponenten möglich ist. Luftzerlegungsanlagen mit Innenverdichtung sind beispielsweise a.a.O., Abschnitt 2.2.5.2, "Internal Compression", erläutert.

Die Destillationssäulensysteme typischer Luftzerlegungsanlagen werden bei unterschiedlichen Betriebsdrücken in ihren Destillationssäulen betrieben. Bekannte Doppelsäulensysteme weisen beispielsweise eine sogenannte Hochdrucksäule (auch lediglich als Drucksäule bezeichnet) und eine sogenannte Niederdrucksäule auf. Der Betriebsdruck der Hochdrucksäule beträgt beispielsweise 4,3 bis 6,9 bar, vorzugsweise etwa 5,0 bar. Die Niederdrucksäule wird bei einem Betriebsdruck von beispielsweise 1,3 bis 1,7 bar, vorzugsweise etwa 1,5 bar betrieben. Der leicht überatmosphärische Betriebsdruck der Niederdrucksäule dient dabei im Wesentlichen dazu, Produkte aus einer entsprechenden Luftzerlegungsanlage ohne die Verwendung zusätzlicher Pumpen entnehmen zu können. Bei den hier und im Folgenden angegebenen Drücken handelt es sich um Absolutdrücke.

In der Hochdrucksäule eines Doppelsäulensystems wird bekanntermaßen aus in die Hochdrucksäule eingespeister Luft ein sauerstoffangereichertes Sumpfprodukt (engl. auch als Enriched Liquid bezeichnet) gewonnen und in die Niederdrucksäule überführt. In der Niederdrucksäule wird aus dem sauerstoffangereicherten Sumpfprodukt aus der Hochdrucksäule und ggf. weiteren, in die Niederdrucksäule eingespeisten Strömen ein überwiegend Sauerstoff enthaltendes Sumpfprodukt abgeschieden. Das Sumpfprodukt der Niederdrucksäule wird, um einen aufsteigenden Gasstrom in der Niederdrucksäule bereitzustellen und damit die Rektifikation aufrechtzuerhalten, kontinuierlich erwärmt, so dass kontinuierlich ein Teil des Sumpfprodukts in die Gasphase übergeht. Die Erwärmung kann in einem innen- oder außenliegenden Kondensatorverdampfer, auch als Hauptkondensator bezeichnet, erfolgen, der mit einem gasförmigen, stickstoffreichen Kopfprodukt der Hochdrucksäule beheizt wird.

Problematisch kann hierbei sein, dass sich bei dem beschriebenen Betrieb der Niederdrucksäule in ihrem Sumpf oder in einem Verdampfungsraum eines entsprechenden außenliegenden Kondensatorverdampfers über die Zeit schwerer flüchtige Komponenten anreichern können, die aus dem sauerstoffangereicherten Sumpfprodukt aus der Hochdrucksäule und damit letztlich aus der eingespeisten Luft sowie den ggf. weiteren, in die Niederdrucksäule eingespeisten Strömen stammen. Als kritisch sind hierbei insbesondere Kohlenwasserstoffe mit bis zu vier Kohlenstoffatomen, aber auch Verbindungen wie Lachgas und Kohlendioxid anzusehen, die mit üblichem Aufwand nicht vollständig aus der Einsatzluft abgetrennt werden konnten.

Maximal zulässige Konzentrationen entsprechender Verbindungen sind beispielsweise im Dokument 65/13/E des Industrial Gases Council (IGC) der European Industrial Gases Association (EIGA), Appendices E und F, "Maximum contaminant levels in liquid oxygen thermosyphon reboiler operation at 1.2 bar abs" und "Maximum contaminant levels in liquid oxygen downflow reboiler operation at 1.2 bar abs", angegeben. Wie dort ferner in Abschnitt 7.3.2, "Purging", ausgeführt, besteht das Problem der Anreicherung weniger in Anlagen, in denen ein signifikanter Anteil flüssiger oder gasförmiger, innenverdichteter sauerstoffreicher Produkte gewonnen wird, weil hier aus dem Sumpf der Niederdrucksäule oder einem Verdampfungsraum eines entsprechenden außenliegenden Kondensatorverdampfers kontinuierlich ein Teil des Sumpfprodukts flüssig abgezogen wird. In Luftzerlegungsanlagen, in denen der Niederdrucksäule jedoch nur bereits gasförmige, sauerstoffreiche Ströme entnommen werden, ist es hingegen erforderlich, vorzugsweise kontinuierlich, einen kleinen Teil des Sumpfprodukts als sogenannte Spülmenge abzuziehen. In der zitierten Publikation der EIGA werden hierbei 0,1 bis 0,2% der eingesetzten Luftmenge vorgeschlagen. Ist eine kontinuierliche Entnahme nicht möglich, soll eine geeignete Menge intermittierend, d.h. mindestens alle 12 Stunden, entnommen werden.

Auch in den Kopfkondensatoren bekannter Luftzerlegungsanlagen mit Destillationssäulen zur Argongewinnung, deren Verdampfungsraum mit einem sauerstoffangereicherten Sumpfprodukt der Hochdrucksäule beschickt wird, kann es zu einer entsprechenden Anreicherung unerwünschter Komponenten kommen. Entsprechendes gilt auch für die Sümpfe von Krypton/Xenon-Anreicherungssäulen, wie unten erläutert. Im Allgemeinen treten entsprechende Probleme immer dann auf, wenn mittels einer tiefkalten sauerstoffreichen Flüssigkeit ein Flüssigkeitsvolumen in einem Verdampfungsraum gespeist und kontinuierlich ein Teil des Flüssigkeitsvolumens durch Verdampfen in die Gasphase überführt wird, insbesondere dann, wenn dem Flüssigkeitsvolumen keine nennenswerten Anteile flüssig entnommen werden.

In der Praxis können sich Schwierigkeiten bei der Einstellung der Menge, die aus entsprechenden Verdampfungsräumen flüssig abgezogen wird, also der Spülmenge, ergeben. So ist es aus ökonomischen Gründen wünschenswert, diese Menge so gering wie möglich zu halten, da diese typischerweise anschließend nicht mehr verwendet werden kann und daher dem Prozess verloren geht. Ferner kommt es beim Ablassen von kryogenen Flüssigkeiten unter Umgehung von Wärmetauschern zwangsläufig zu Kälteverlusten. Andererseits reichern sich bei einer zu geringen Menge die erwähnten Komponenten übermäßig in den Verdampfungsräumen an.

Es besteht daher der Bedarf nach einer einfachen und zuverlässigen Möglichkeit, die Anreicherung von schwerer als Sauerstoff siedenden Komponenten in einer sauerstoffreichen, tiefkalten Flüssigkeit zu ermitteln, so dass eine einfache und kostengünstige Möglichkeit zur Verfügung steht, die Einhaltung von Vorgaben für entsprechend zu entnehmende Mengen überprüfen und/oder einstellen zu können.

### Offenbarung der Erfindung

Diese Aufgabe wird durch ein Verfahren zur Tieftemperaturzerlegung von Luft und eine entsprechende Luftzerlegungsanlage gemäß den Oberbegriffen der unabhängigen Patentansprüche gelöst. Bevorzugte Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1 %, 0,1% oder 0,01% auf molarer, Gewichts- oder Volumenbasis stehen kann. Der Begriff "überwiegend" kann der Definition von "reich" entsprechen. Flüssige und gasförmige Ströme können im hier verwendeten Sprachgebrauch ferner angereichert oder abgereichert an einer oder mehreren Komponenten sein, wobei sich diese Begriffe im Fall der Luftzerlegung auf die eingesetzte Luft bzw. deren Bestandteile beziehen. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf den jeweiligen Gehalt in der eingesetzten Luft, enthält. Ist hier von "flüssigem Sauerstoff' die Rede, sei darunter ein flüssiger Strom verstanden, der reich an Sauerstoff ist, jedoch nicht ausschließlich aus Sauerstoff bestehen muss.

In Luftzerlegungsanlagen werden an unterschiedlichen Stellen tiefkalte Flüssigkeiten erhalten und in Verdampfungsräumen unter Aufrechterhaltung vorgegebener Füllstände kontinuierlich verdampft. Dies ist beispielsweise in dem eingangs erläuterten Sumpf der Niederdrucksäule der Fall, der zugleich einen Verdampfungsraum eines Hauptkondensators darstellt. Ein entsprechender Hauptkondensator kann selbstverständlich auch außerhalb eines Doppelsäulensystems angeordnet sein, er wird in diesem Fall als außenliegender Hauptkondensator bezeichnet. Die vorliegende Erfindung kommt in beliebigen Verdampfungsräumen zum Einsatz, insbesondere in Verdampfungsräumen von Kondensatorverdampfern. Kennzeichnend für einen Verdampfungsraum ist, dass in ihm mittels einer tiefkalten Flüssigkeit ein Flüssigkeitsvolumen gebildet und aufrechterhalten wird, von welchem kontinuierlich ein Teil durch Verdampfen in die Gasphase überführt wird. Ein "Kondensatorverdampfer" weist einen Verflüssigungsraum und einen Verdampfungsraum auf. Verdampfungs- und Verflüssigungsraum werden jeweils durch Gruppen von Passagen (Verflüssigungs- bzw. Verdampfungspassagen) gebildet, die untereinander in fluidischer Verbindung stehen. In dem Verflüssigungsraum wird die Kondensation eines ersten Fluidstroms durchgeführt, in dem Verdampfungsraum die Verdampfung eines zweiten Fluidstroms. Die beiden Fluidströme stehen dabei in indirektem Wärmetausch.

Unter einer "tiefkalten" Flüssigkeit, bzw. einem entsprechenden Fluid, flüssigen Luftprodukt, Strom usw. wird ein flüssiges Medium verstanden, dessen Siedepunkt deutlich unterhalb der jeweiligen Umgebungstemperatur, beispielsweise bei weniger als 200 K, insbesondere bei 77 K bis 110 K, liegt. Beispiele für tiefkalte Medien sind flüssige Luft, flüssiger Sauerstoff und flüssiger Stickstoff im obigen Sinn.

### Vorteile der Erfindung

Die vorliegende Erfindung geht von einem an sich bekannten Verfahren zur Tieftemperaturzerlegung von Luft aus, bei dem in einem Destillationssäulensystem einer Luftzerlegungsanlage ein in einem Verdampfungsraum vorliegendes Flüssigkeitsvolumen mittels einer tiefkalten Flüssigkeit gespeist und bei dem kontinuierlich ein Teil des Flüssigkeitsvolumens durch Verdampfen in die Gasphase überführt wird. Die tiefkalte Flüssigkeit, insbesondere eine sauerstoffangereicherte Flüssigkeit aus dem Sumpf einer Niederdrucksäule, enthält neben Sauerstoff schwerer als Sauerstoff siedende Komponenten, darunter Xenon. Insbesondere kann eine entsprechende Flüssigkeit im Rahmen der zuvor getroffenen Definition überwiegend aus Sauerstoff bestehen. Selbstverständlich können aber auch höher als Sauerstoff siedende Komponenten enthalten sein. Der Gehalt an Xenon richtet sich nach dem Xenongehalt der natürlichen Luft, die über den Erdball im Wesentlichen konstant ist. Xenon wird von üblichen Aufreinigungseinrichtungen für Einsatzluft in Luftzerlegungsanlagen nicht zurückgehalten und stört die Trennung nicht weiter.

Hingegen richtet sich der Gehalt der wenigstens einen weiteren schwerer als Sauerstoff siedenden Komponente unter anderem nach dem Betriebsort einer entsprechenden Luftzerlegungsanlage. Wie in dem zuvor zitierten EIGA-Dokument, insbesondere Abschnitt 7.4, "Contaminant Analysis", angegeben, ist ein erhöhter Gehalt derartiger Komponenten insbesondere in hochindustrialisierten Gebieten mit chemischen, petrochemischen und metallurgischen Anlagen zu erwarten. Je nach Art der Industrie können hier beispielsweise erhöhte Mengen Lachgas, Kohlenwasserstoffe und/oder Kohlendioxid zu verzeichnen sein. Insbesondere Kohlendioxid wird zwar in üblichen Aufreinigungseinrichtungen der Einsatzluft weitgehend entzogen, Restgehalte können jedoch in ein entsprechendes Destillationssäulensystem eingespeist werden.

Die vorliegende Erfindung schlägt vor, den Gehalt an Xenon in dem Flüssigkeitsvolumen zu bestimmen und als Maß für eine Anreicherung der schwerer als Sauerstoff siedenden Komponenten in dem Flüssigkeitsvolumen zu verwenden.

Die Erfindung kann dabei umfassen, Xenon als Maß für eine Konzentration wenigstens einer der schwerer als Sauerstoff siedenden Komponenten in einem entsprechenden Flüssigkeitsvolumen einzusetzen. Es kann also auf Grundlage des in dem Flüssigkeitsvolumen bestimmten Gehalts an Xenon ein Gehalt an wenigstens einer der schwerer als Sauerstoff siedenden Komponenten bestimmt werden. Dies hat, wie auch nachfolgend erläutert, den Vorteil, dass nur der Gehalt einer Komponente, nämlich Xenon, bestimmt werden muss und die Konzentration einer oder mehrerer Komponenten auf dieser Grundlage zumindest größenordnungsmäßig abgeschätzt werden kann. Die Messung anderer, insbesondere mehrerer unterschiedlicher, schwerer als Sauerstoff siedender Komponenten, ggf. mit unterschiedlichen Analysatoren, ist in diesem Fall nicht mehr erforderlich.

Die Erfindung ist aber auch insbesondere von Vorteil in Fällen, in denen lediglich die Anreicherung selbst ermittelt wird. So sind in den vorliegend betrachteten Verdampfungsräumen, beispielsweise in einem Sumpf einer Niederdrucksäule oder einem Verdampfungsraum eines Kopfkondensators einer Rohargonsäule (siehe unten) nicht notwendigerweise Flussmesseinrichtungen vorgesehen, weil es ausgesprochen aufwendig ist, kleine Ströme an kryogenen Flüssigkeiten zu messen. Typischerweise eingesetzte Ultraschallsonden weisen konstruktionsbedingt Spalte auf, die insbesondere im Bereich von hochangereichertem Sauerstoff unerwünscht sind, da sich gerade dort eine Anreicherung von Kohlenwasserstoffen ergeben könnte. Die diesen Verdampfungsräumen entnommenen Spülmengen werden daher herkömmlicherweise häufig über die verwendete Ventilgröße und die Entnahmezeit bzw. die Frequenz der Entnahme vergleichsweise grob abgeschätzt. Der erfindungsgemäß vorgeschlagene Einsatz von Xenon als Maß für die Anreicherung der schwerer als Sauerstoff siedenden Komponenten erleichtert die Bestimmung der korrekten Spülmenge beträchtlich. Im Rahmen der vorliegenden Erfindung kann es ausreichend sein, die Spülmenge bei zu hoher Anreicherung entsprechend zu erhöhen und das Ergebnis anhand einer weiteren Messung zu bestätigen.

Es kann also im Rahmen der vorliegenden Erfindung auch vorgesehen sein, auf Grundlage des Maßes für die Anreicherung eine Flussrate zumindest eines flüssig dem Flüssigkeitsvolumen entnommenen Stroms zu ermitteln. Die Ermittlung einer solchen Flussrate erfolgt damit indirekt, so dass auf die Verwendung aufwendiger Flussmesseinrichtungen verzichtet werden kann.

Die Verwendung von Xenon hat sich im Rahmen der vorliegenden Erfindung als besonders vorteilhaft herausgestellt, da Xenon, wie erwähnt, in der Außenluft in im Wesentlichen konstanter Konzentration auftritt. Ferner ist Xenon so wenig flüchtig, dass es bei der Verdampfung übriger Komponenten, insbesondere von Sauerstoff, aus dem Flüssigkeitsvolumen im Wesentlichen in diesem verbleibt, d.h. nicht oder allenfalls geringfügig in die Gasphase übergeht. Letzteres ist insbesondere nicht bei anderen, theoretisch ebenfalls als Marker bzw. Indikatoren verwendbaren Komponenten wie Krypton und Argon der Fall.

Geht man in einem entsprechenden Flüssigkeitsvolumen, beispielsweise bei einer Entnahme von ausschließlich gasförmigen Sauerstoffprodukten aus einer Niederdrucksäule, von einem typischen Anreicherungsfaktor von 500 aus, beträgt die minimale Xenonkonzentration in dem Flüssigkeitsvolumen das 500-fache der Konzentration in der Luft, also 500 x 0,086 vppm und damit 43 vppm. Eine derartige Konzentration ist mittels bekannter Messeinrichtungen, wie unten erläutert, vergleichsweise einfach messbar.

Wird also in einem Flüssigkeitsvolumen eines entsprechenden Verdampfungsraums im erläuterten Beispiel ein Gehalt von 43 vppm Xenon gemessen, kann umgekehrt davon ausgegangen werden, dass in dem Verdampfungsraum eine 500-fache Anreicherung von Xenon erfolgt ist, die Spülmenge also 1/500 der eingesetzten Luftmenge betragen hat. Die Erfindung erlaubt also die sichere Bestimmung der Anreicherung ohne eine insbesondere bei tiefkalten Temperaturen aufwendige Flussmessung.

Da der Gehalt an Xenon in der Umgebungsluft bekannt ist und die Gehalte anderer, schwerer als Sauerstoff siedender Komponenten wie Lachgas und Kohlendioxid mit ausreichender Sicherheit größenordnungsmäßig abgeschätzt werden können, ist es möglich, aus dem Xenongehalt in dem Flüssigkeitsvolumen Rückschlüsse auf den Gehalt an den weiteren schwerer als Sauerstoff siedenden Komponenten zu ziehen.

Eine größenordnungsmäßige Bestimmung kann dabei ausreichend sein. Wird beispielsweise ein Wert von ca. 2 vppm Methan oder ca. 0,32 vppm Lachgas in der eingesetzten Luft angenommen, was in der überwiegenden Mehrzahl der Fälle zutrifft, und legt der bestimmte Gehalt an Xenon in dem Flüssigkeitsvolumen eine 1.000-fache Anreicherung nahe, wird der Maximalwert für Methan 2.000 vppm betragen, vorausgesetzt, dass kein Methan aus dem Flüssigkeitsvolumen verdampft. Dies ist beispielsweise in Anordnungen mit sogenannten Sperrböden der Fall, wie sie bei der Gewinnung von Krypton und Xenon zum Einsatz kommen. Geht 1/3 des Methans in die Gasphase über, beträgt der tatsächliche Gehalt in dem Flüssigkeitsvolumen hingegen nur 1.300 vppm. Bei einer maximal zulässigen Konzentration von 500 vppm Methan bedeutet dies in jedem Fall, dass zu viel Methan vorhanden ist. Entsprechendes gilt möglicherweise auch für andere störende Luftkomponenten. Eine separate Messung von Methan und anderen störenden Luftkomponenten wie Lachgas und Kohlendioxid ist jedoch nicht erforderlich.

In der EP 0 726 434 B1 wurde vorgeschlagen, als Marker Lachgas zu verwenden. Lachgas ist jedoch in größeren Mengen in entsprechenden Flüssigkeitsvolumina als kritisch anzusehen, da es bei tiefen Temperaturen Mischkristalle mit Kohlendioxid bilden kann, die Passagen der Wärmetauscher verlegen können. Im Ergebnis kann dies zu einem erhöhten Druckabfall und zur indirekten Anreicherung von Kohlenwasserstoffen in Bereichen geringerer Strömung führen.

Der besondere Vorteil der Verwendung von Xenon, wie sie erfindungsgemäß vorgeschlagen wird, besteht, wie bereits teilweise erwähnt, darin, dass Xenon weder von Molekularsieb- noch von Flüssigadsorbern, also typischen, in Luftzerlegungsanlagen eingesetzten Reinigungseinrichtungen, zurückgehalten wird. Xenon, das in vergleichsweise einfach nachweisbaren Konzentrationen in der eingesetzten Luft enthalten ist, geht damit vollständig in ein entsprechendes Flüssigkeitsvolumen über. Außerdem ist Xenon ausreichend schwer, um vollständig in dem Flüssigkeitsvolumen zu verbleiben, d.h. es erfolgt beispielsweise kein Austrag über ein gasförmiges Sauerstoffprodukt. Keine andere Komponente, die natürlicherweise in einem entsprechenden Flüssigkeitsvolumen einer Luftzerlegungsanlage enthalten ist, weist diese Eigenschaften auf.

Das erfindungsgemäße Verfahren entfaltet besondere Vorteile in einer Luftzerlegungsanlage, wie sie eingangs erläutert wurde, nämlich einer Luftzerlegungsanlage mit einem Destillationssäulensystem, das eine Hochdrucksäule und eine Niederdrucksäule umfasst. In diesem Fall wird das Flüssigkeitsvolumen in einem Verdampfungsraum eines die Hochdrucksäule und die Niederdrucksäule wärmetauschend verbindenden Kondensatorverdampfers, des erläuterten Hauptkondensators, verdampft. Gespeist wird dieses Flüssigkeitsvolumen mit sauerstoffangereicherter Flüssigkeit aus der Hochdrucksäule. Die Erfindung ist besonders vorteilhaft, wenn eine entsprechende Luftzerlegungsanlage nicht zur Innenverdichtung von Sauerstoff eingerichtet ist, d.h. wenn der Sauerstoff der Niederdrucksäule lediglich in Form eines oder mehrerer sauerstoffangereicherter gasförmiger Ströme entnommen wird.

Die vorliegende Erfindung eignet sich jedoch auch in besonderer Weise für Luftzerlegungsanlagen mit Säulen zur Argongewinnung, so beispielsweise für Luftzerlegungsanlagen, die eine Rohargonsäule umfassen. In diesem Fall wird das Flüssigkeitsvolumen in einem Verdampfungsraum des Kopfkondensators der Rohargonsäule verdampft. Um zu vermeiden, dass sich in entsprechenden Flüssigkeitsvolumina unerwünscht hohe Konzentrationen störender Komponenten anreichern, wird typischerweise ein Anteil hiervon über eine Leitung abgezogen und kontinuierlich in die Niederdrucksäule überführt. Da dies jedoch aus den erläuterten Gründen typischerweise nicht strömungsüberwacht erfolgt, kann beispielsweise bei einer (auch teilweisen) Verlegung einer entsprechenden Leitung eine übermäßige Anreicherung unerwünschter Komponenten erfolgen. Durch die erfindungsgemäße Bestimmung des Gehalts an Xenon kann dies erkannt und durch Einleiten geeigneter Maßnahmen verhindert werden. In entsprechender Weise eignet sich die vorliegende Erfindung aber auch für die Bestimmung von schwerer als Sauerstoff siedenden Komponenten in Kopfkondensatoren von Reinargonsäulen oder für beliebige andere zusätzliche Verdampfungsräume in entsprechenden Kondensatoren oder anderen Verdampfungseinrichtungen. In allen Fällen wird das Flüssigkeitsvolumen vorteilhafterweise aus einem Fluid gebildet, das flüssig aus der Hochdrucksäule abgezogen und in den entsprechenden Verdampfungsraum überführt wird.

Besondere Vorteile werden im Rahmen der vorliegenden Erfindung jedoch auch erzielt, wenn der Gehalt an Xenon im Sumpf einer Krypton/Xenon-Anreicherungssäule als Verdampfungsraum bestimmt wird. Entsprechende Anreicherungssäulen sind aus der einschlägigen Fachliteratur, beispielsweise H.-W. Häring (Hrsg.), Industrial Gases Processing, Wiley-VCH, 2006, insbesondere Abschnitt 3.3, "Recovery of Krypton and Xenon", bekannt. Sie werden typischerweise aus dem Sumpf der Niederdrucksäule, also ihrerseits aus einem Flüssigkeitsvolumen eines Verdampfungsraums, gespeist.

Im Sumpf einer Anreicherungssäule werden Krypton und Xenon typischerweise um den Faktor 2.000 bis 3.000 angereichert. Eine Bestimmung des Gehalts an Xenon an dieser Stelle gibt nicht nur Auskunft über die Qualität des weiter zu verarbeitenden Produkts (d.h. den tatsächlichen Anreicherungsfaktor), sondern auch über die Methankontamination der Außenluft. Methan wird nicht von Molekularsiebadsorbern zurückgehalten. Ist daher der Xenongehalt im Sumpf der Anreicherungssäule gering (beispielsweise 43 vppm, was, wie erwähnt, auf eine 500-fache Anreicherung rückschließen lässt), aber die Konzentration an Kohlenwasserstoffen unerwartet hoch, so ist die Außenluftkonzentration an Methan nicht spezifikationsgerecht, d.h. eine außergewöhnlich hohe Luftverschmutzung liegt vor. Der Gehalt an Kohlenwasserstoffen ist in diesem speziellen Fall bekannt, da die Anreicherungssäule auf Grundlage des Gesamtkohlenwasserstoffgehalts betrieben wird, der hierzu in geeigneter Weise überwacht wird.

Auch die Gehalte an Lachgas und Kohlendioxid werden im Sumpf der Anreicherungssäule typischerweise überwacht. Treten übermäßig hohe Konzentrationen an Kohlendioxid (über 2 vppm) und/oder Lachgas (über 50 vppm) auf, und liegt die Xenonkonzentration gleichzeitig im normalen Bereich, so ist davon auszugehen, dass ein Fehlbetrieb der Molekularsiebadsorber vorliegt. Beispielsweise kann ein unerwünschter Luftbypass vorliegen. Ein entsprechender Fehlbetrieb ist an anderer Stelle innerhalb der Luftzerlegungsanlage deutlich schwerer nachzuweisen.

Zusammengefasst sieht die vorliegende Erfindung also vorteilhafterweise vor, einen Gehalt an wenigstens einem Kohlenwasserstoff in dem Verdampfungsraum zu bestimmen, mit dem Gehalt an Xenon in Beziehung zu setzen und als Maß für eine Reinheit der in der Luftzerlegungsanlage zerlegten Luft zu verwenden und/oder einen Gehalt an Kohlendioxid und/oder Lachgas in dem Verdampfungsraum zu bestimmen, mit dem Gehalt an Xenon in Beziehung zu setzen und zur Ermittlung der Funktionsfähigkeit wenigstens einer Luftreinigungseinrichtung der Luftzerlegungsanlage zu verwenden. Auf diese Weise erlaubt die Bestimmung des Gehalts an Xenon im Sumpf der Anreicherungssäule also eine indirekte Überwachung der Außenluftkonzentration auf erhöhte Kohlenwasserstoffkontaminationen und gleichzeitig eine Überwachung des Betriebs der Molekularsiebadsorber.

Wie eingangs erläutert, wird vorteilhafterweise aus entsprechenden Verdampfungsräumen kontinuierlich oder periodisch ein Anteil des Flüssigkeitsvolumens abgezogen, welcher 0,1 bis 1 Volumenprozent der zur Gewinnung der tiefkalten Flüssigkeit verwendeten Luft beträgt. Auf diese Weise kann die übermäßige Anreicherung entsprechender Komponenten sicher verhindert werden. Die Erfindung ist besonders vorteilhaft, wenn der Anteil des Flüssigkeitsvolumens, der kontinuierlich oder periodisch aus dem Verdampfungsraum abgezogen wird, auf Grundlage des Gehalts an Xenon, der, wie erwähnt, als Maß für einen Gehalt an der wenigstens einen weiteren schwerer als Sauerstoff siedenden Komponente in der tiefkalten Flüssigkeit verwendet wird, eingestellt wird. Die vorliegende Erfindung kann dabei im Rahmen eines Regelverfahrens implementiert werden, das beispielsweise aufgrund des Gehalts an Xenon den abzuziehenden Anteil automatisch regelt.

Für die Bestimmung des Xenongehalts in der zuvor erläuterten Konzentration eignet sich insbesondere die Verwendung der Gaschromatographie, insbesondere wenn hierbei ein Wärmeleitfähigkeitsdetektor (WLD, engl. Thermal Conductivity Detector, TCD) verwendet wird. Entsprechende Chromatographieverfahren sind einfach, robust und kostengünstig durchführbar.

Die vorliegende Erfindung kann entweder in einer Luftzerlegungsanlage oder mittels einer separat zur Luftzerlegungsanlage bereitgestellten Messeinrichtung implementiert werden. Beispielsweise kann zur Messung des Gehalts an Xenon eine periodisch messende Messeinrichtung der Luftzerlegungsanlage verwendet werden oder es kann zur Messung des Gehalts an Xenon eine Messprobe aus dem Verdampfungsraum entnommen und in einem Probenbehälter zur Messeinrichtung überführt werden. Geeignete Probenbehälter sind beispielsweise Probenzylinder oder für den Transport von Gasen spezifisch ausgebildete Beutel aus mit Kunststoff beschichteten Metallfolien. Eine Messung muss also nicht vor Ort erfolgen.

Eine Luftzerlegungsanlage, die zur Tieftemperaturzerlegung von Luft in einem Destillationssäulensystem eingerichtet ist, ist ebenfalls Gegenstand der vorliegenden Erfindung. Eine derartige Luftzerlegungsanlage umfasst Mittel, die dafür eingerichtet sind, ein in dem Destillationssäulensystem in einem Verdampfungsraum vorliegendes Flüssigkeitsvolumen mittels einer tiefkalten Flüssigkeit zu speisen und kontinuierlich einen Teil des Flüssigkeitsvolumens durch Verdampfen in die Gasphase zu überführen, wobei die tiefkalte Flüssigkeit neben Sauerstoff schwerer als Sauerstoff siedende Komponenten, darunter Xenon, enthält. Erfindungsgemäß sind Mittel vorgesehen, die dafür eingerichtet sind, einen Gehalt von Xenon in dem Flüssigkeitsvolumen zu bestimmen und als ein Maß für eine Anreicherung der schwerer als Sauerstoff siedenden Komponenten in dem Flüssigkeitsvolumen zu verwenden.

Eine derartige Luftzerlegungsanlage, zu deren Merkmalen und Vorteilen ausdrücklich auf die zuvor erläuterten Merkmale und Vorteile verwiesen wird, ist insbesondere zur Durchführung eines Verfahrens eingerichtet, wie es zuvor erläutert wurde, und weist die entsprechenden Mittel auf.

Die Erfindung wird nachfolgend unter Bezugnahme auf die beigefügte Zeichnung näher erläutert, welche eine Luftzerlegungsanlage zeigt, anhand derer die erfindungsgemäßen Maßnahmen erläutert werden.

### Kurze Beschreibung der Zeichnung

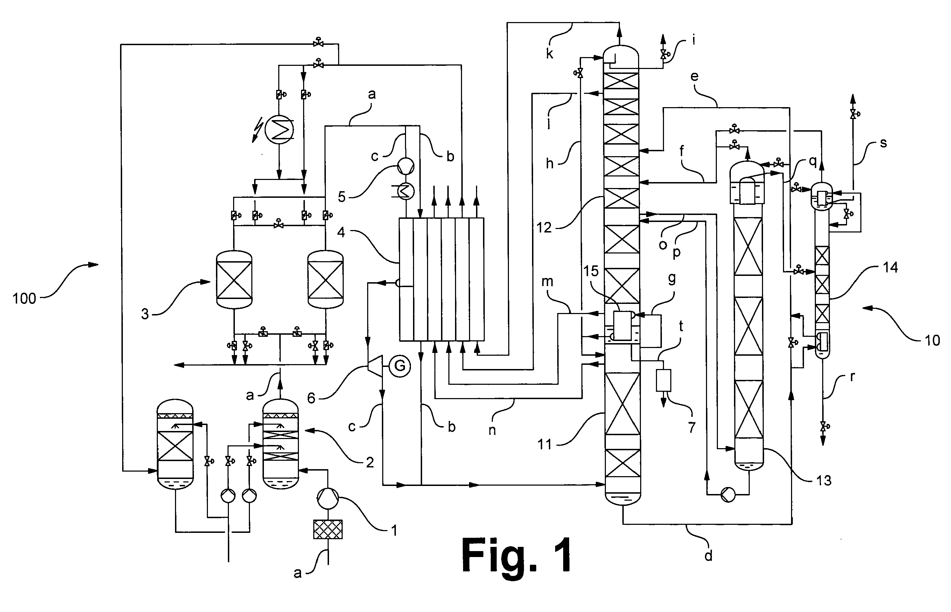

Figur 1 zeigt eine Luftzerlegungsanlage in Form eines schematischen Prozessflussdiagramms.

### Ausführliche Beschreibung der Zeichnung

In Figur 1 ist eine Luftzerlegungsanlage dargestellt, anhand derer eine Ausführungsform der Erfindung veranschaulicht wird. Die Luftzerlegungsanlage ist insgesamt mit 100 bezeichnet.

In der Luftzerlegungsanlage 100 wird ein Einsatzluftstrom a mittels eines Hauptluftverdichters 1 über einen Filter (ohne Bezeichnung) angesaugt und verdichtet. Der entsprechend verdichtete Einsatzluftstrom a wird einer mit Kühlwasser betriebenen und hier nicht näher erläuterten Vorkühleinrichtung 2 zugeführt. Der vorgekühlte Einsatzluftstrom, weiterhin mit a bezeichnet, wird in einer Reinigungseinrichtung 3 aufgereinigt. In der Reinigungseinrichtung 3, die typischerweise ein Paar von im Wechselbetrieb eingesetzten Adsorberbehältern (Molekularsiebadsorbern) umfasst, wird der vorgekühlte Einsatzluftstrom dabei weitgehend, jedoch prinzipbedingt nicht völlig, von Wasser und Kohlendioxid befreit.

Stromab der Reinigungseinrichtung 3 wird der Einsatzluftstrom, auch hier noch mit a bezeichnet, in zwei Teilströme b und c aufgeteilt. Der Teilstrom b wird auf dem Druckniveau des Einsatzluftstroms a in einem Hauptwärmetauscher 4 abgekühlt. Der Teilstrom c wird in einem Nachverdichter 5 nachverdichtet und ebenfalls, jedoch nur auf ein Zwischentemperaturniveau, in dem Hauptwärmetauscher 4 abgekühlt. Dieser Teilstrom c, der sogenannte Turbinenstrom, wird nach der Abkühlung auf das Zwischentemperaturniveau hier mittels einer Generatorturbine 6 auf das Druckniveau des Teilstroms b entspannt, mit letzterem vereinigt, und in eine Hochdrucksäule 11 eines unten im Detail erläuterten Destillationssäulensystems 10 eingespeist.

In der Hochdrucksäule 11 des Destillationssäulensystems 10 wird aus der über die Teilströme b und c eingespeisten Einsatzluft eine sauerstoffangereicherte flüssige Sumpffraktion sowie eine stickstoffangereicherte gasförmige Kopffraktion gebildet. Die sauerstoffangereicherte flüssige Sumpffraktion wird als Strom d aus der Hochdrucksäule 11 abgezogen, teilweise als Heizmedium in einem Sumpfverdampfer einer Reinargonsäule 14 (siehe unten) verwendet und jeweils in definierten Anteilen in einen Kopfkondensator der Reinargonsäule 14, einen Kopfkondensator einer Rohargonsäule 13 sowie, als Strom e, in eine Niederdrucksäule 12 des Destillationssäulensystems 10 eingespeist. In den Verdampfungsräumen der Kopfkondensatoren der Rohargonsäule 13 und der Reinargonsäule 14 verdampfendes Fluid wird ebenfalls, als Strom f, in die Niederdrucksäule 12 überführt.

Vom Kopf der Hochdrucksäule 11 kann das gasförmige stickstoffreiche Kopfprodukt in Form des Stroms g abgezogen, in einem Hauptkondensator 15, der eine wärmetauschende Verbindung zwischen der Hochdrucksäule 11 und der Niederdrucksäule 12 herstellt, verflüssigt, und in Anteilen als Rücklauf auf die Hochdrucksäule 11 aufgegeben und in die Niederdrucksäule 12 entspannt werden.

Aus einer Flüssigkeitsrückhalteeinrichtung am Kopf der Niederdrucksäule 12 kann ein flüssiger stickstoffreicher Strom i abgezogen und als Flüssigstickstoffprodukt aus der Luftzerlegungsanlage 100 ausgeführt werden. Ein vom Kopf der Niederdrucksäule 12 abgezogener gasförmiger stickstoffreicher Strom k wird durch den Hauptwärmetauscher 4 geführt und als Stickstoffprodukt auf dem Druck der Niederdrucksäule bereitgestellt. Aus der Niederdrucksäule 12 wird ferner ein Strom I aus einem oberen Bereich abgezogen und nach Erwärmung in dem Hauptwärmetauscher 4 als sogenannter Unreinstickstoff in der Vorwärmeinrichtung 2 bzw. nach einer Aufheizung mittels eines elektrischen Heizers in der Reinigungseinheit 3 verwendet.

Ein sauerstoffreicher gasförmiger Strom m wird aus einem unteren Bereich der Niederdrucksäule 12 abgezogen und ebenfalls nach Erwärmung in dem Hauptwärmetauscher 4 als entsprechendes Sauerstoffprodukt bereitgestellt. Aus einem oberen Bereich der Hochdrucksäule 11 wird ein stickstoffreicher Strom n entnommen, in dem Hauptwärmetauscher 4 erwärmt und als gasförmiges Druckstickstoffprodukt auf dem Druck der Hochdrucksäule 11 bereitgestellt.

Man erkennt, dass in der Luftzerlegungsanlage 100, wie sie in Figur 1 veranschaulicht ist, im Sumpfbereich der Niederdrucksäule 12 zwar Flüssigkeit auskondensiert wird, dem in diesem Verdampfungsraum vorliegenden Flüssigkeitsvolumen jedoch normalerweise keine Flüssigkeit entnommen wird. Da in der dargestellten Luftzerlegungsanlage 100 Sauerstoff lediglich in Form des gasförmigen Stroms m aus einem unteren Bereich der Niederdrucksäule 12 entnommen wird, nicht jedoch, wie in bekannten Innenverdichtungsverfahren, flüssiger Sauerstoff, kann es im Sumpf der Niederdrucksäule daher zur Anreicherung schwerer als Sauerstoff siedender Komponenten kommen.

Zur Gewinnung von Reinargon wird der Niederdrucksäule 12 am bekannten Argonübergang oder knapp darunter ein Strom o entnommen und in die bereits erwähnte Rohargonsäule 13 überführt. Ein im Sumpf der Rohargonsäule 13 anfallendes Kondensat wird in Form des Stroms p in die Niederdrucksäule 12 zurückgepumpt. Am Kopf der Rohargonsäule 13 nicht im Kopfkondensator auskondensierendes Fluid wird in Form des Stroms q abgezogen und in die Reinargonsäule 14 überführt. In der Reinargonsäule 14 wird unter Verwendung des bereits erwähnten Sumpfverdampfers und des ebenfalls erwähnten Kopfkondensators Flüssigargon r gewonnen. Ein gewisser Anteil verlässt die Reinargonsäule als Strom s und wird in die Atmosphäre abgeblasen.

Man erkennt, dass in den Kopfkondensatoren der Rohargonsäule 13 und der Reinargonsäule 14, ähnlich wie im Sumpf der Niederdrucksäule 12 zwar Fluide anfallen bzw. hier flüssige Ströme eingespeist werden, jedoch keine flüssigen Ströme entnommen werden. Auch hier kann es daher zu der bereits erwähnten Anreicherung von schwerer siedenden Komponenten kommen. Eine typischerweise vorgesehene Entnahme von Fluid zur Verhinderung einer entsprechenden Anreicherung ist in der Figur 1 nicht veranschaulicht.

Die vorliegende Erfindung schlägt daher, wie bereits mehrfach erläutert, beispielsweise vor, in entsprechenden Verdampfungsräumen, worunter insbesondere die Verdampfungsräume im Sumpf der Niederdrucksäule 12, im Kopfkondensator der Rohargonsäule 13 und im Kopfkondensator der Reinargonsäule 14 verstanden werden, enthaltene Flüssigkeitsvolumina auf ihre Xenongehalte zu überprüfen und bei Überschreiten eines vorgegebenen Werts entsprechende Maßnahmen einzuleiten.

Hierzu sind im dargestellten Beispiel ein Strom t und eine Einrichtung 7 veranschaulicht. Bei dem Strom t handelt es sich um einen intermittierend dem Flüssigkeitsvolumen im Verdampfungsraum der Niederdrucksäule entnommenen Anteil, der in der Einrichtung 7 auf seinen Xenongehalt überprüft wird. Wie mehrfach erläutert, kann die Überprüfung beispielsweise mittels Gaschromatographie erfolgen. Die Überprüfung muss nicht vor Ort, d.h. in der Luftzerlegungsanlage 100 selbst, erfolgen, es kann auch vorgesehen sein, den dem Verdampfungsraum der Niederdrucksäule entnommenen Anteil in einer externen Einrichtung auf seinen Xenongehalt zu überprüfen.

## Patentansprüche

1. Verfahren zur Tieftemperaturzerlegung von Luft, bei dem in einem Destillationssäulensystem (10) einer Luftzerlegungsanlage (100) ein in einem Verdampfungsraum vorliegendes Flüssigkeitsvolumen mittels einer tiefkalten Flüssigkeit gespeist wird und kontinuierlich ein Teil des Flüssigkeitsvolumens durch Verdampfen in die Gasphase überführt wird, wobei die tiefkalte Flüssigkeit neben Sauerstoff schwerer als Sauerstoff siedende Komponenten, darunter Xenon, enthält, **dadurch gekennzeichnet, dass** der Gehalt an Xenon in dem Flüssigkeitsvolumen bestimmt und als Maß für eine Anreicherung der schwerer als Sauerstoff siedenden Komponenten in dem Flüssigkeitsvolumen verwendet wird.

2. Verfahren nach Anspruch 1, bei dem auf Grundlage des in dem Flüssigkeitsvolumen bestimmten Gehalts an Xenon ein Gehalt an wenigstens einer der schwerer als Sauerstoff siedenden Komponenten bestimmt wird.

3. Verfahren nach Anspruch 1 oder 2, bei dem auf Grundlage des Maßes für die Anreicherung eine Flussrate zumindest eines flüssig dem Flüssigkeitsvolumen entnommenen Stroms ermittelt wird.

4. Verfahren nach einem der vorstehenden Ansprüche, bei dem eine Luftzerlegungsanlage (100) mit einem Destillationssäulensystem (10) verwendet wird, das eine Hochdrucksäule (11) und eine Niederdrucksäule (12) umfasst.

5. Verfahren nach Anspruch 4, bei dem der Verdampfungsraum, in dessen Flüssigkeitsvolumen der Gehalt an Xenon bestimmt wird, ein Verdampfungsraum eines die Hochdrucksäule (11) und die Niederdrucksäule (12) wärmetauschend verbindenden Hauptkondensators (15) ist.

6. Verfahren nach Anspruch 4 oder 5, bei dem eine Luftzerlegungsanlage (100) mit einer Rohargonsäule (13) verwendet wird und der Verdampfungsraum, in dessen Flüssigkeitsvolumen der Gehalt an Xenon bestimmt wird, ein Verdampfungsraum eines Kopfkondensators der Rohargonsäule (13) ist.

7. Verfahren nach einem der Ansprüche 4 bis 6, bei dem eine Luftzerlegungsanlage mit einer Reinargonsäule (14) verwendet wird und der Verdampfungsraum, in dessen Flüssigkeitsvolumen der Gehalt an Xenon bestimmt wird, ein Verdampfungsraum eines Kopfkondensators der Reinargonsäule (14) ist.

8. Verfahren nach einem der Ansprüche 4 bis 7, bei dem die tiefkalte Flüssigkeit aus einem Fluid gebildet wird, das flüssig aus der Hochdrucksäule (11) abgezogen und anschließend in den Verdampfungsraum überführt wird.

9. Verfahren nach einem der vorstehenden Ansprüche, bei dem eine Luftzerlegungsanlage (100) mit einem Destillationssäulensystem (10) verwendet wird, das eine Krypton/Xenon-Anreicherungssäule umfasst, wobei der Verdampfungsraum, in dessen Flüssigkeitsvolumen der Gehalt an Xenon bestimmt wird, der Sumpf der Krypton/Xenon-Anreicherungssäule ist.

10. Verfahren nach Anspruch 9, bei dem ferner ein Gehalt an wenigstens einem Kohlenwasserstoff in dem Verdampfungsraum bestimmt, mit dem Gehalt an Xenon in Beziehung gesetzt und als Maß für eine Reinheit der in der Luftzerlegungsanlage (100) zerlegten Luft verwendet wird.

11. Verfahren nach Anspruch 9 oder 10, bei dem ferner ein Gehalt an Kohlendioxid und/oder Lachgas in dem Verdampfungsraum bestimmt, mit dem Gehalt an Xenon in Beziehung gesetzt und zur Ermittlung der Funktionsfähigkeit wenigstens einer Luftreinigungseinrichtung der Luftzerlegungsanlage (100) verwendet wird.

12. Verfahren nach einem der vorstehenden Ansprüche, bei dem kontinuierlich oder intermittierend ein Anteil des Flüssigkeitsvolumens aus dem Verdampfungsraum abgezogen wird, der 0,1 bis 1 Volumenprozent der zur Gewinnung der tiefkalten Flüssigkeit verwendeten Luft entspricht.

13. Verfahren nach Anspruch 12, bei dem der Anteil des Flüssigkeitsvolumens, das kontinuierlich oder periodisch aus dem Verdampfungsraum abgezogen wird, auf Grundlage des Gehalts an Xenon eingestellt wird.

14. Verfahren nach einem der vorstehenden Ansprüche, bei dem der Gehalt an Xenon mittels Gaschromatographie, insbesondere unter Verwendung eines Leitfähigkeitsdetektors, bestimmt wird.

15. Luftzerlegungsanlage (100), die zur Tieftemperaturzerlegung von Luft in einem Destillationssäulensystem (10) eingerichtet ist, mit Mitteln, die dafür eingerichtet sind, ein in einem Verdampfungsraum des Destillationssäulensystems (10) vorliegendes Flüssigkeitsvolumen mittels einer tiefkalten Flüssigkeit zu speisen und kontinuierlich einen Teil des Flüssigkeitsvolumens durch Verdampfen in die Gasphase zu überführen, wobei die tiefkalte Flüssigkeit neben Sauerstoff schwerer als Sauerstoff siedende Komponenten, darunter Xenon, enthält, **dadurch gekennzeichnet, dass** Mittel vorgesehen sind, die dafür eingerichtet sind, einen Gehalt an Xenon in dem Flüssigkeitsvolumen zu bestimmen und als ein Maß für eine Anreicherung der schwerer als Sauerstoff siedenden Komponenten in dem Flüssigkeitsvolumen zu verwenden.
